# EUROPEAN PATENT APPLICATION

(11) **EP 3 799 894 A1**
(43) Date of publication of application: **07.04.2021**
(21) Application number: 19201309.2
(22) Date of filing: 03.10.2019
(51) Int. Cl.: A61L 33/00, A61B 90/70, A61B 50/10, A61B 50/13

(54) **SYSTEM FOR TRANSPORTING WASHED MEDICAL EQUIPMENT AND PACKING THE WASHED MEDICAL EQUIPMENT IN CONTAINERS IN A STERILE PROCESSING DEPARTMENT**

(71) Applicant: Gibotech A/S, 5220 Odense SØ (DK)
(72) Inventor: ANKER, Henrik, 5220 Odense SØ (DK)
(74) Representative: AWA Denmark A/S

(57) **Abstract**

Disclosed is a system for transporting washed medical equipment and packing the washed medical equipment in containers in a sterile processing department. The system comprises: a packing table comprising a first working position for a human operator; an automated washing machine for washing medical equipment; and one or more autonomous transport vehicles for transporting medical equipment to the packing table. The system further comprises a receiving element for receiving a container comprising washed medical equipment, the receiving element being arranged in connection with the packing table, the receiving element being movable between a receiving position and a working position. An autonomous transport vehicle is configured to transport a container comprising washed medical equipment from the automated washing machine to the receiving element, and the receiving element being configured to receive a container comprising washed medical equipment by moving from the working position to the receiving position.

## Description

### Field

The present disclosure relates to a system for transporting washed medical equipment and packing the washed medical equipment in containers in a sterile processing department, and to a sterile processing department comprising such a system.

### Background

Increasing healthcare cost is a global problem which every country faces. Automation of manual process in hospitals can be part of the solution. However, due the high complexity of hospitals the degree of atomization until now has been low.

Cleaning and sterilizing of medical equipment such as endoscopes and surgical instruments in a sterile processing department is an example of a work intensive process. Today used medical equipment is typically provided to the sterile processing department in containers and manually moved e.g. on a trolley to a pre-washing table. At the pre-washing table the medical equipment is manually pre-washed. The pre-washed medical equipment is then arranged in a new container and the new container is manually moved to a washing machine. Next, the washed medical equipment is arranged in a sterilization container at a packing table and manually moved to an autoclave for sterilization. The process involves the use of a significant number of different containers having different dimensions.

WO2017077073 discloses a system that uses transport vehicles for automating part of the processes in a sterile processing department. However, the disclosed transport vehicles are complex and may induce discomfort on the humans working in the sterile processing department as they may travel in proximity of the primary working positions of the humans. Furthermore, the high number of transport vehicles needed for transporting and temporarily storing the equipment may result in traffic jams.

Thus it remains a problem to provide a simpler system that can aid in automating processes in a sterile processing department and further improve the working environment for the human operators.

### Summary

According to a first aspect, the disclosure relates to a system for transporting washed medical equipment and packing the washed medical equipment in containers in a sterile processing department, the system comprises:
- a packing table comprising a first working position for a human operator;
- an automated washing machine for washing medical equipment; and
- one or more autonomous transport vehicles for transporting medical equipment to the packing table;
wherein the system further comprises a receiving element for receiving a container comprising washed medical equipment, the receiving element being arranged in connection with the packing table, the receiving element being movable between a receiving position at a first height and a working position at a second height, wherein one of the one or more autonomous transport vehicle is configured to transport a container comprising washed medical equipment from the automated washing machine to the receiving element, and the receiving element being configured to receive a container comprising washed medical equipment by moving from the working position to the receiving position.

Consequently, by providing the system with a receiving element, the receiving element being movable between a receiving position at a first height and a working position at a second height, wherein the receiving element is configured to receive a container at the receiving position, the receiving element may be adjusted to the height of the transport vehicle. The height of the transport vehicle may therefore be lowered, whereby a simpler and more stable transport vehicle may be provided. The transport vehicle may furthermore be provided without height adjustment capabilities. A further advantage of having a receiving element is that the working area around the user working at the washing station is kept more secure, because the transport vehicle is kept substantially away from the user. This is in contrast to known systems. The receiving element provides therefore a more secure system by keeping the elements of the system at a substantially constant distance from the user, which thereby do not have to worry about e.g. the transport vehicle driving around him.

The autonomous transport vehicle comprises a drive mechanism and travelling means such as wheels or chain-wheels. The autonomous transport vehicle may comprise navigation means such as sensors and/or cameras for navigation in order to orientate itself and such that it doesn't collide with any other object. The autonomous transport vehicle may be guided with guiding means such as floor stripes e.g. colored stripes or magnetic stripes. The guiding means may also be vision, or laser means.

The transport vehicle may follow markers or wires in the floor, lasers or any other guiding means for navigation. The transport vehicle may also be fully configured to travel without any guiding means e.g. by having a preprogrammed travelling path or by having a map of the navigation area for determining the travelling path based on the map and the navigation means. The transport vehicle may comprise a motorized roller, belt, or chain conveyor for delivering the container.

The automated washing machine may comprise a buffer storage for storing containers with wash medical equipment before they are being picked-up by an autonomous transport vehicle. Alternatively, the autonomous driving vehicle may also support the container in the automated washing machine.

The receiving position is typically lower than the working position. The receiving element may be configured to automatically return to the working position after having received the container comprising the washed medical equipment. The receiving element may comprise a motorized roller, belt, or chain conveyor for receiving a container with medical equipment from an autonomous transport vehicle and/or for delivering an empty container to an autonomous transport vehicle. A container may be transferred e.g. by the conveyor of the transport vehicle, by the conveyor of the receiving element, or by the combination of the conveyor of the transport vehicle and the conveyor of the receiving element. The receiving element may comprise a support element secured to the floor and a movable element movable relative to the support element between the receiving position and the working position. The receiving element may comprise an actuator configured to move the movable element e.g. a linear actuator and / or a motor. The receiving element may be configured to receive a container comprising washed medical equipment by moving from the working position to the receiving position, by firstly at the receiving position delivering an empty container to a first autonomous transport vehicle, and secondly receiving the container comprising washed medical equipment from a second autonomous transport vehicle.

The packing table may be height adjustable. The packing table may have a table lamp allowing the human operator to inspect the washed medical equipment before it is packed. The packing table may further be provided with a working station configured to allow the human operator to register the medical equipment before packing it e.g. by scanning an ID of the medical equipment e.g. a barcode or a QR-code.

The container may be a sterilizing container e.g. a container configured to support the medical equipment in an autoclave. The sterilizing container may be provided with a lit allowing a sterilizing fluid e.g. hot steam, to enter the container. The lid may further be configured to form a fluid tight seal when the sterilizing container cools down whereby the medical equipment within the medical container may be stored under sterile conditions.

Alternatively, the medical equipment may be packed in sterilizing pouches and / or sterilizing paper and placed in the container. The container may still support the packed medical equipment in the autoclave but it is the sterilizing pouches and / or sterilizing paper that secures the sterile conditions after the autoclave treatment.

In some embodiments, one of the one or more the autonomous transport vehicles comprises one or more motorized conveyor elements for loading and or unloading a container for medical equipment.

In some embodiments, the one or more motorized conveyor elements are a plurality of motorized convoy rolls.

In some embodiments, the receiving element further comprises one or more motorized conveyor elements for loading and / or unloading a container for medical equipment.

In some embodiments, one of the one or more autonomous transport vehicles is configured to receive an empty container for medical equipment from the receiving element and transport the empty container away from the packing table, the receiving element being configured to deliver the empty container to the one autonomous transport vehicle by activating the one or more motorized conveyor elements after the autonomous transport vehicle has been aligned with the receiving element.

The receiving element may be configured to move from the working position to the receiving position, when the autonomous transport vehicle that is to transport the empty container away approaches e.g. by receiving a control signal from a central control unit and / or a control signal directly from the autonomous transport vehicle.

In some embodiments, the system further comprises an outgoing element arranged in connection with the packing table, the outgoing element being configured to deliver a container comprising washed medical equipment to one of the one or more autonomous transport vehicles, the outgoing element being movable between an outgoing position at a first height and a working position at a second height, wherein the outgoing element being configured to deliver the container by moving from the working position to outgoing position and wherein the one autonomous transport vehicle is configured to transport the container away from the packing table.

The outgoing position is typically lower than the working position. The outgoing element may be configured to automatically return to the working position after having delivered the container comprising the packed and inspected medical equipment. The outgoing element may comprise a motorized roller, belt, or chain conveyor for delivering the container to an autonomous transport vehicle and/or for receiving an empty container to an autonomous transport vehicle. A container may be transferred e.g. by the conveyor of the transport vehicle, by the conveyor of the receiving element, or by the combination of the conveyor of the transport vehicle and the conveyor of the receiving element. The outgoing element may comprise a support element secured to the floor and a movable element movable relative to the support element between the delivery position and the working position. The outgoing element may comprise an actuator configured to move the movable element e.g. a linear actuator and / or a motor. The outgoing element may receive an empty container from an autonomous transport vehicle before returning to the working position.

In some embodiments, the outgoing element has a first and a second area, where the container is arranged at first area and another container is arranged at the second area, wherein the first area is provided with first one or more motorized conveyor elements and the second area is provided with second one or more conveyor elements, the first and second one or more motorized conveyor elements being configured to deliver a container to an autonomous transport vehicle, and wherein the first one or more motorized conveyor elements is controllable independent of the second one or more motorized conveyer elements.

In some embodiments, the outgoing element comprises a central support supporting the first area and the second area, the first area and the second area extend out from the central support, and wherein the central support is movable between a first position and a second position and configured to move the first area together with the second area between the outgoing position and the working position.

Consequently, the outgoing element may be design simpler. Furthermore, by having the first and second area extending from the central support, the outgoing element may function like a table allowing the human operator to closer to the outgoing element.

In some embodiments, the packing table has a first edge extending along a longitudinal axis, the human operator facing the first edge when working at the packing table, the system having a primary working zone abutting the first edge, the primary working zone extending at least 2 meters along the longitudinal axis and 1.5 meters along a first axis, the first axis being orthogonal to the longitudinal axis and laying in the horizontal plane, and wherein the system is configured so to that none of the one or more autonomous vehicles enters the primary working zone.

Consequently, the working environment of the human operator may be improved as he / she may feel comfortable knowing that the zone they will spend most of their working time is free from autonomous transport vehicles.

In some embodiments, the packing table has a first end and a second end opposite to the first end, the first edge extending form the first end to the second end, the receiving element being arranged around the first end, the receiving element having a first end and a second end opposite to the first end, the receiving element being configured to receive the container comprising washed medical equipment at the first end from the autonomous transport vehicle, and wherein the primary working zone extends along the longitudinal axis at least from the first end of the receiving element until the second end of the packing table.

In some embodiments, the outgoing element is being arranged around the second end of the packing table, the outgoing element having a first end and a second end opposite to the first end, the outgoing element being configured to deliver the container comprising washed medical equipment at the first end, and wherein the primary working zone extends along the longitudinal axis from the first end of the receiving element until the first end of the outgoing element.

In some embodiments, the one or more autonomous transport vehicles are configured to stay out of the primary working zone.

In some embodiments, the one or more autonomous transport vehicles are configured to stay out of the primary working zone by being provided with a positioning system and / or by detecting markers in the floor.

In some embodiments, the system further comprises an input unit and a control unit, the input unit being communicatively coupled to the control unit, the control unit having access to a database, the database storing a plurality of user profiles and wherein the input unit is configured to allow a human operator to select a user profile of the plurality of user profiles and wherein the user profile sets the working position of the receiving element, the outgoing element and / or lightning conditions.

Consequently, the system may be adapted to the human operator thereby securing a good ergonomic working environment.

The user profile may additionally set the working height of the packing table.

The lightning condition may be lightning conditions within the primary working zone. The lightning conditions may set lightning parameters for one or more table lamps arranged at the packing table and / or one or more celling lamps configured to provide lightning to the primary working zone. The lightning conditions may be brightness of the light, color temperature of the light and / or color of the light.

In some embodiments, the receiving position of the receiving element is lower the working position of the receiving element, and wherein the one or more transport vehicles are only capable of moving a container in the horizontal plane.

In some embodiments, the system comprises a plurality of packing tables each provided with a receiving element, and a plurality of autonomous transport vehicles, wherein the plurality of autonomous transport vehicles are configured to move along a main route, the main route passes each of the plurality of packing tables and both starts and ends at the automated washing machine, and wherein the plurality of autonomous transport vehicles are configured to travel along the main route in a single direction only.

Consequently, the travel time for the autonomous transport vehicles may be easier to estimate and the risk of traffic jams may be lowered. This may further reduce the space requirements for the main route.

According to a second aspect the disclosure relates to use of a system disclosed in relation to the first aspect of the disclosure in a sterile processing department.

According to a third aspect the disclosure relates to a sterile processing department comprising a system disclosed in relation to the first aspect of the disclosure.

The different aspects of the present invention can be implemented in different ways including a transport system, use of a transport system and a sterile processing department described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependant claims. Furthermore, it will be appreciated that embodiments described in connection with one of the aspects described herein may equally be applied to the other aspects.

### Brief description of the drawings

The above and/or additional objects, features and advantages of the present invention, will be further elucidated by the following illustrative and nonlimiting detailed description of embodiments of the present invention, with reference to the appended drawings, wherein:
Figs. 1a-b show a schematic drawing of a system according to an embodiment.
Figs. 2 shows show a schematic drawing of a system according to an embodiment.
Fig. 3 shows a receiving element according to an embodiment.
Fig. 4 shows an autonomous transport vehicle according to an embodiment.
Figs. 5a-b show an outgoing element according to an embodiment.
Fig. 6 shows a schematic drawing of a system according to an embodiment.

### Detailed description

In the following description, reference is made to the accompanying figures, which show by way of illustration how the invention may be practiced.

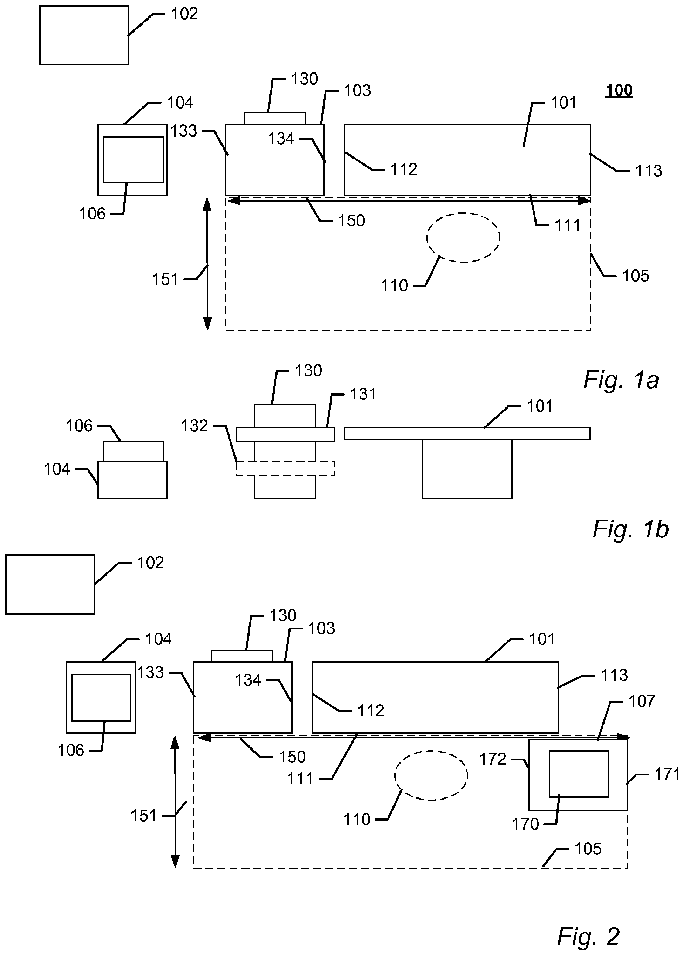

Figs. 1a-b show a schematic drawing of a system 100 for transporting washed medical equipment and packing the washed medical equipment in containers in a sterile processing department according to an embodiment, where Fig. 1a shows a top view and Fig. 1b shows a side view. The system comprises a packing table 101 comprising a first working position for a human operator 110, an automated washing machine 102 for washing medical equipment, one or more autonomous transport vehicles 104 for transporting medical equipment to the packing table, and a receiving element 103 for receiving a container 106 comprising washed medical equipment. The automated washing machine 102 is in the drawing arranged relative close to packing table 101. However, this has been done only for illustrative purpose. In reality the automated washing machine will often be arranged further away from the packing table 100 e.g. at least 10, 20 or 30 meters. The receiving element 103 is arranged in connection with the packing table 101 and movable between a receiving position 132 at a first height and a working position 131 at a second height. The autonomous transport vehicle 104 is configured to transport a container 106 comprising washed medical equipment from the automated washing machine 102 to the receiving element 104. The autonomous transport vehicle 104 may be controlled by a central control unit. The automated washing machine 102 may deliver containers comprising washed medical equipment to a buffer storage before the containers are being picked-up by an autonomous transport vehicle 104. The receiving element 104 is configured to receive a container comprising washed medical equipment by moving from the working position 131 to receiving position 132. The receiving element 103 may receive a control signal from a central control unit signaling to the receiving element 103 that it should move the receiving position 132 when the autonomous transport vehicle 104 approaches. Alternatively, the receiving element 103 may communicate directly with the autonomous transport vehicle 104, i.e. the autonomous transport vehicle 104 may send a control signal to the receiving element when it approaches. By having a receiving element 103 arranged in connection with the packing table 101 a large primary working zone 105 may be formed where the autonomous transport vehicles do not enter. This will improve the working conditions for the human operator as the human operator may move around within the primary working zone 105 without fearing to collide with autonomous transport vehicles that may potentially may hold sharp medical instruments. Furthermore, by using a receiving element that can move up and down, simple and safe autonomous transport vehicles with a low center of gravity may be used.

The packing table has a first edge 111 extending along a longitudinal axis 150, the human operator 110 facing the first edge 111 when working at the packing table 101, the primary working zone 105 abutting the first edge 111. The primary working zone 105 may extend at least 2 meters along the longitudinal axis 150 and 1.5 meters along a first axis 151, the first axis 151 being orthogonal to the longitudinal axis 150 and laying in the horizontal plane.

The packing table has a first end 112 and a second end 113 opposite to the first end 112, the first edge 111 extending form the first end 112 to the second end 113, the receiving element 103 being arranged around the first end 112 (e.g. closer to the first end 112 than the second end 113). The receiving element 103 has a first end 133 and a second end 134 opposite to the first end 133, the receiving element 103 being configured to receive the container 106 comprising washed medical equipment at the first end 133 from the autonomous transport vehicle 104. In this embodiment the primary working zone 105 extends along the longitudinal axis 150 at least from the first end of receiving element 133 until the second end of the packing table 113.

Fig. 2 shows a schematic drawing of a system 100 for transporting washed medical equipment and packing the washed medical equipment in containers in a sterile processing department according to an embodiment. The system comprises a packing table 101 comprising a first working position for a human operator 110, an automated washing machine 102 for washing medical equipment, one or more autonomous transport vehicles 104 for transporting medical equipment to the packing table, and a receiving element 103 for receiving a container 106 comprising washed medical equipment. The packing table 101, the automated washing machine 102, the one or more autonomous transport vehicles 104, and the receiving element 103 are the same as shown in Fig. 1a-b. However, the system comprises further an outgoing element 107 arranged in connection with the packing table 101, the outgoing element 107 being configured to deliver a container 170 comprising washed medical equipment to one of the one or more autonomous transport vehicles 104. The outgoing element 107 being movable between an outgoing position at a first height and a working position at a second height. The outgoing element 107 being configured to deliver the container 170 by moving from the working position to outgoing position. The one or more autonomous transport vehicles 104 being configured to transport the container 170 away from the packing table 101. The container 170 may be a container storing medical equipment packed in sterilizing pouches and / or sterilizing paper, where the medical equipment is packed at the packing table 101. Alternatively, the container 170 may be a sterilizing container provided with a lit allowing a sterilizing fluid e.g. hot steam, to enter the container.

In this embodiment, the outgoing element 107 is arranged around the second end 113 of the packing table 101, the outgoing element 107 has a first end 171 and a second end 172 opposite to the first end 171, the outgoing element being configured to deliver the container 170 at the first end 171. The primary working zone 105 extends along the longitudinal axis 150 from the first end 133 of the receiving element until the first end 171 of the outgoing element. Consequently, an even larger primary working zone 105 may be formed.

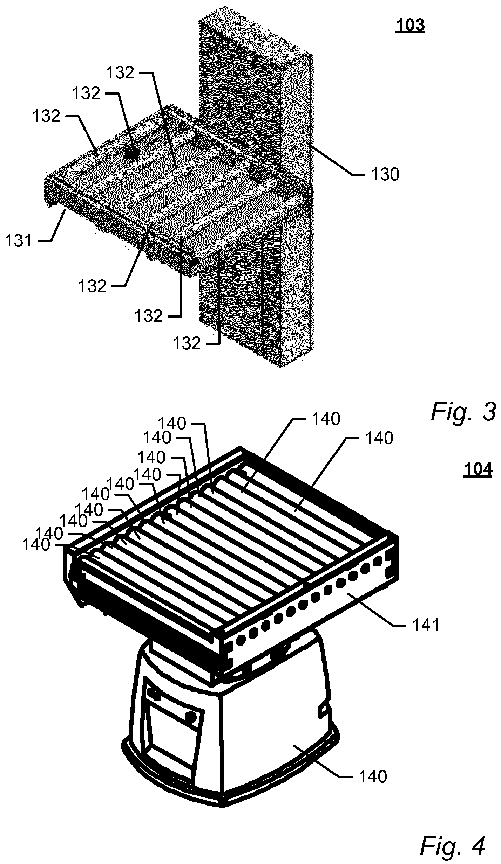

Fig. 3 shows a perspective view of a receiving element 103, according to an embodiment. The receiving element 103 has a support element 130 secured to the floor and a movable element 131 arranged in connection with the support element 130. The receiving element 103 has an actuator (not shown) configured to move the movable element 131 between a receiving position and a working position. The movable element 131 has a plurality of motorized conveyor rolls 132.

Fig. 4 shows a perspective view of an autonomous transport vehicle 104, according to an embodiment. The autonomous transport vehicle 104 has a lower element 140 and an upper element 141. The lower element 140 has a drive mechanism (not shown) allowing the transport vehicle 104 to travel. The upper element 141 is arranged on top of the lower element 140. The upper element 140 has a plurality of motorized conveyor rolls 140 for delivering and receiving containers with medical equipment.

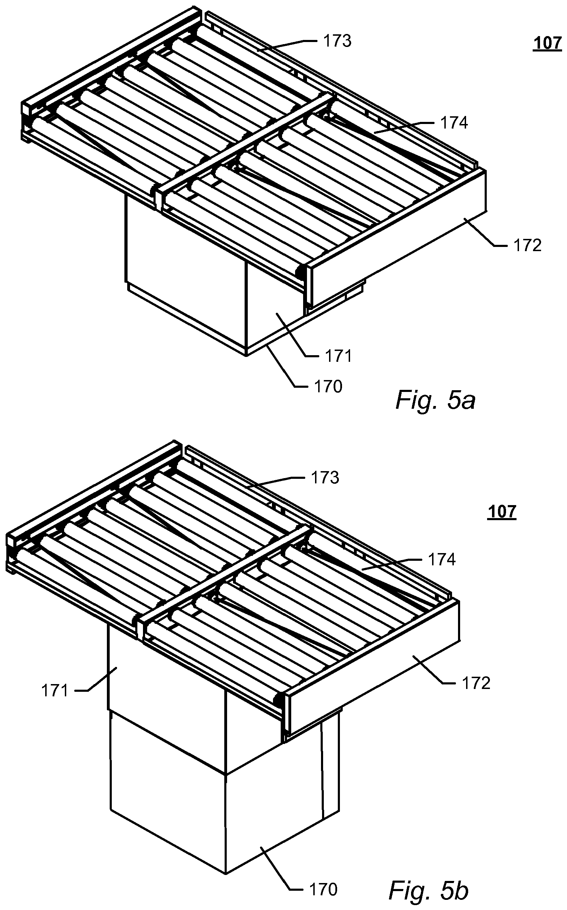

Figs. 5a-b show a perspective view of an outgoing element 107, according to an embodiment. Fig. 5a shows the outgoing element 107 in a delivering position and Fig. 5b shows the outgoing element 107 in a working position. The outgoing element 107 has a central support 170 171 and a top element 172 arranged on top of the central support 170 171. The central support 170 171 has a first part 170 secured to a floor and a second 171 movable relative to the first part. The top element 172 has a plurality of motorized conveyor rollers for delivering and receiving containers. The receiving element 107 has a first area 173 and a second area 174. Each of the first area 173 and the second area 174 have conveyor rollers. The receiving element 107 may thereby have two containers simultaneously, one on the first area 173 and one on the second area 174.

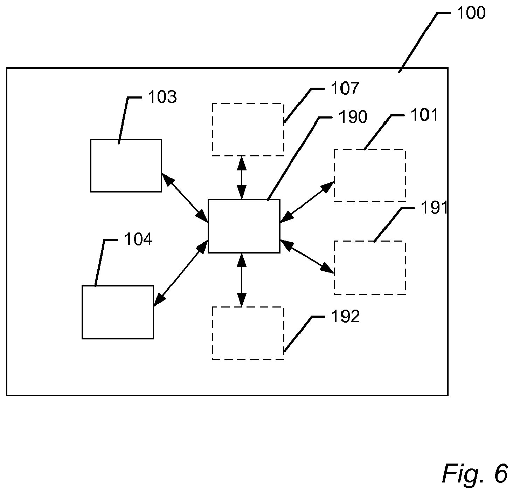

Fig. 6 shows a schematic drawing of a system 100 for transporting washed medical equipment and packing the washed medical equipment in containers in a sterile processing department according to an embodiment. The system may be similar to the system disclosed in relation to Figs. 1 or 2. The system comprises central control unit 190 communicatively coupled to a receiving element 103 and an autonomous transport vehicle 104. The central control unit is configured to send control signal to the receiving element 103 e.g. to control the receiving element 103 to move between the receiving position and the working position. Correspondingly, the central control unit 190 is configured to send control signals to the autonomous transport vehicle 103 to control the autonomous transport vehicle to move to a particular position or active transport elements e.g. motorized conveyor rolls of the autonomous transport vehicle. The system may further comprise an outgoing element 107 communicatively coupled to the central control unit 190, where the central control unit is configured to send control signal to the outgoing element 107 e.g. to control the outgoing element 107 to move between the delivering position and the working position. The central control unit may further be communicatively coupled to an input unit 191 and a database 192, where the database 192 stores a plurality of user profiles and wherein the input unit 191 is configured to allow a human operator to select a user profile of the plurality of user profiles and wherein the user profile sets the working position of the receiving element 103 and / the outgoing element 107.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A system for transporting washed medical equipment and packing the washed medical equipment in containers in a sterile processing department, the system comprises:
• a packing table comprising a first working position for a human operator;
• an automated washing machine for washing medical equipment; and
• one or more autonomous transport vehicles for transporting medical equipment to the packing table;
wherein the system further comprises a receiving element for receiving a container comprising washed medical equipment, the receiving element being arranged in connection with the packing table, the receiving element being movable between a receiving position at a first height and a working position at a second height, wherein one of the one or more autonomous transport vehicle is configured to transport a container comprising washed medical equipment from the automated washing machine to the receiving element, and the receiving element being configured to receive a container comprising washed medical equipment by moving from the working position to the receiving position.

2. A system according to claim 2, wherein one of the one or more the autonomous transport vehicles comprises one or more motorized conveyor elements for loading and or unloading a container for medical equipment.

3. A system according to any one of the preceding claims, wherein the receiving element further comprises one or more motorized conveyor elements for loading and / or unloading a container for medical equipment.

4. A system according to claim 4, wherein one of the one or more autonomous transport vehicles is configured to receive an empty container for medical equipment from the receiving element and transport the empty container away from the packing table, the receiving element being configured to deliver the empty container to the one autonomous transport vehicle by activating the one or more motorized conveyor elements after the autonomous transport vehicle has been aligned with the receiving element.

5. A system according to any one of the preceding claims, wherein the system further comprises an outgoing element arranged in connection with the packing table, the outgoing element being configured to deliver a container comprising washed medical equipment to one of the one or more autonomous transport vehicles, the outgoing element being movable between an outgoing position at a first height and a working position at a second height, wherein the outgoing element being configured to deliver the container by moving from the working position to outgoing position and wherein the one autonomous transport vehicle is configured to transport the container away from the packing table.

6. A system according to claim 5, wherein the outgoing element has a first and a second area, where the container is arranged at first area and another container is arranged at the second area, wherein the first area is provided with first one or more motorized conveyor elements and the second area is provided with second one or more conveyor elements, the first and second one or more motorized conveyor elements being configured to deliver a container to an autonomous transport vehicle, and wherein the first one or more motorized conveyor elements is controllable independent of the second one or more motorized conveyer elements.

7. A system according to claim 6, wherein the outgoing element comprises a central support supporting the first area and the second area, the first area and the second area extend out from the central support, and wherein the central support is movable between a first position and a second position and configured to move the first area together with the second area between the outgoing position and the working position.

8. A system according to any one of claims 1 to 7, wherein the packing table has a first edge extending along a longitudinal axis, the human operator facing the first edge when working at the packing table, the system having a primary working zone abutting the first edge, the primary working zone extending at least 2 meters along the longitudinal axis and 1.5 meters along a first axis, the first axis being orthogonal to the longitudinal axis and laying in the horizontal plane, and wherein the system is configured so to that none of the one or more autonomous vehicles enters the primary working zone.

9. A system according to claim 8, wherein the packing table has a first end and a second end opposite to the first end, the first edge extending form the first end to the second end, the receiving element being arranged around the first end, the receiving element having a first end and a second end opposite to the first end, the receiving element being configured to receive the container comprising washed medical equipment at the first end from the autonomous transport vehicle, and wherein the primary working zone extends along the longitudinal axis at least from the first end of the receiving element until the second end of the packing table.

10. A system according to claim 9 when dependent on claims 5 to 7, wherein the outgoing element is being arranged around the second end of the packing table, the outgoing element having a first end and a second end opposite to the first end, the outgoing element being configured to deliver the container comprising washed medical equipment at the first end, and wherein the primary working zone extends along the longitudinal axis from the first end of the receiving element until the first end of the outgoing element.

11. A system according to any one of claims 9 to 11, wherein the one or more autonomous transport vehicles are configured to stay out of the primary working zone.

12. A system according to claim 11, wherein the one or more autonomous transport vehicles are configured to stay out of the primary working zone by being provided with a positioning system and / or by detecting markers in the floor.

13. A system according to any one of claims 1 to 12, wherein the system further comprises an input unit and a control unit, the input unit being communicatively coupled to the control unit, the control unit having access to a database, the database storing a plurality of user profiles and wherein the input unit is configured to allow a human operator to select a user profile of the plurality of user profiles and wherein the user profile sets the working position of the receiving element, the outgoing element and / or lightning conditions.

14. A system according to any one of claims 1 to 13, wherein the system comprises a plurality of packing tables each provided with a receiving element, and a plurality of autonomous transport vehicles, wherein the plurality of autonomous transport vehicles are configured to move along a main route, the main route passes each of the plurality of packing tables and both starts and ends at the automated washing machine, and wherein the plurality of autonomous transport vehicles are configured to travel along the main route in a single direction only.

15. A sterile processing department comprising a system according to any one of claims 1 to 14.
